# EUROPÄISCHE PATENTSCHRIFT

(11) **EP 2 415 422 B1**
(45) Veröffentlichungstag und Bekanntmachung des Hinweises auf die Patenterteilung: **01.08.2018**
(21) Anmeldenummer: 11176604.4
(22) Anmeldetag: 04.08.2011
(51) Int. Cl.: A61F 2/26

(54) **Zylinder für eine Penisprothese mit entfernbarem Führungsmittel**
Cylinder for a penis prosthetic with removable guidance
Cylindre pour une prothèse de pénis dotée d'un moyen de guidage pouvant être enlevé

(30) Priorität: 05.08.2010 DE 102010038975
(43) Veröffentlichungstag der Anmeldung: 08.02.2012
(73) Patentinhaber: ET Elastomer Technik GmbH, 97762 Hammelburg (DE)
(72) Erfinder: Lakhani, Mukund, 71717 Beilstein (DE); König, Jochen, 74626 Bretzfeld (DE)
(74) Vertreter: Maikowski & Ninnemann Patentanwälte Partnerschaft mbB

(56) Entgegenhaltungen:
- EP-A1- 0 072 167
- EP-A2- 0 137 752
- WO-A1-2011/035787
- DE-A1- 3 524 988
- US-A1- 2004 010 244
- US-A1- 2004 225 182
- US-A1- 2011 071 345
- US-B1- 7 985 176

## Beschreibung

Die Erfindung betrifft einen Zylinder für eine Penisprothese nach dem Oberbegriff des Anspruchs 1.

Die Implantation von Penisprothesen ist eine bekannte Methode zur Behandlung von erektiler Dysfunktion bei männlichen Patienten. Die Penisprothese wird hierbei dazu verwendet, eine künstliche Erektion bei den Patienten zu erzeugen, was ihnen ermöglicht, sexuell wieder aktiv zu werden.

Üblicherweise besteht eine solche Prothese aus einem oder zwei mit Fluid befüllbaren, z. B. aufpump- oder aufblasbaren Zylindern, die in das Corpus cavernosum penis, also in den Penisschwellkörper des Patienten implantiert werden. Dieser bzw. diese Zylinder sind mittels Leitungen mit einer Ventile aufweisenden Pumpeinrichtung fluidtechnisch verbunden, die ihrerseits mit einem Reservoir für eine Kochsalzlösung oder eine andere biokompatible Flüssigkeit gekoppelt ist. Wird die Pumpeinrichtung betätigt, wird in dem Reservoir enthaltenes Fluid, üblicherweise eine Flüssigkeit, von dem Reservoir durch die Leitungen in den oder die Zylinder gepumpt. Ein flexibler Zylinder wird somit unter Druck gesetzt und versteift sich unter diesem aufgebauten Druck. Die gewünschte Erektion wird erzeugt.

Will der Patient wieder in den nicht-erigierten Zustand wechseln, muss das Fluid von den Zylindern wieder in das Reservoir gebracht werden. Hierzu wird üblicherweise ein Ventil der Pumpeinrichtung betätigt und/oder die Zylinder werden komprimiert.

Eine derartige Penisprothese mit zwei gattungsgemäßen Zylindern ist beispielsweise in der Patentschrift US-A 4,566,446 oder in der EP 0 072 167 A2 gezeigt.

Alle Penisprothesen mit Fluid befüllbaren Zylindern haben das Problem, dass der oder die Zylinder in den Corpus cavernosum penis eingesetzt und darin in einer bestimmten Lage angeordnet werden müssen. Dabei ist insbesondere darauf zu achten, dass sich keine Hohlräume zwischen einem Zylinderende und dem umliegenden Gewebe ausbilden. Ein Ende des Zylinders muss genau am Ende des Corpus cavernosum penis so platziert werden, dass er in Richtung der Glans penis, also der Eichel, am Gewebe anliegt, um den sicheren Betrieb der Penisprothese gewährleisten zu können.

In der Praxis wird diese Platzierung eines Zylinders üblicherweise dadurch erreicht, dass mittels eines Fadens, der an dem Ende des Zylinders befestigt ist, der bereits in den Penis eingesetzte Zylinder in eine bestimmungsgemäße Position in dem Corpus cavernosum penis gezogen wird. Hierfür ist es erforderlich, eine Operationsnadel in das lose Ende des Zylinderfadens einzufädeln, die Glans penis vom Penisinneren bzw. dem Corpus cavernosum penis aus zu durchstechen und im Anschluss daran, den Faden durch das so entstandene Loch in der Glans penis zu ziehen, um den Zylinder bestimmungsgemäß in dem Corpus cavernosum penis bzw. der darin gebildeten Kavität zu positionieren.

Der Zylinderfaden wird zwar nach der erfolgreichen Implantation so dicht wie möglich an der Glans penis abgetrennt. Da es jedoch nicht möglich ist, den Zylinderfaden an dem Zylinderende selbst abzutrennen, verbleibt ein Teil des Zylinderfadens im Inneren der Glans penis des Patienten. Der dauerhafte Verbleib des Zylinderfadens in dem Penis ist zwar regelmäßig medizinisch unkritisch, jedoch gerade aus Patientensicht nicht wünschenswert. Darüber hinaus empfinden die Patienten den erforderlichen Einstich an dieser exponierten Stelle als sehr störend und bis zur Genesung schmerzintensiv.

Aus der US 2004/0225182 A1 ist ein Zylinder für eine Penisprothese bekannt, bei dem eine Spitzes eines Führungsmittels in Form eines Führungsstabes in einer Mulde an einer äußeren Zylinderwandung des Zylinders aufgenommen ist, um den Zylinder in eine bestimmungsgemäße Position innerhalb des Penis zu drücken. Nach der Positionierung kann der Führungsstab aus der Mulde gezogen werden. Der Führungsstab ist während des Positionierens des Zylinders mit dem Zylinder lediglich lokal im Bereich der Stabspitze verbunden und ansonsten frei relativ zu dem Zylinder beweglich. Es besteht somit das Risiko, dass über den Führungsstab sowohl bei der Positionierung des Zylinders als auch nach dem Entfernen der Stabspitze aus der Mulde umliegendes Gewebe beschädigt wird.

Die nachveröffentlichte WO 2011/035787 A1 offenbart ferner einen Zylinder für eine Penisprothese nach dem Oberbegriff des Anspruchs 1.

Es ist Aufgabe der vorliegenden Erfindung, einen Zylinder für eine Penisprothese und ein Verfahren zur operativen Implantierung eines solchen Zylinders insoweit zu verbessern, dass die Implantierung des Zylinders erleichtert und eine Verletzung von Gewebe minimiert wird.

Diese Aufgabe wird mit dem Zylinder des Anspruchs 1 gelöst.

So ist ein Zylinder für eine Penisprothese vorgesehen, der in den Penis eines Patienten implantierbar und zur Erzeugung einer Erektion des Penis mit Fluid befüllbar ist, wobei erfindungsgemäß ferner vorgesehen ist, dass der implantierbare Zylinder ein Führungsmittel zur bestimmungsgemäßen Positionierung des Zylinders in dem Penis aufweist. Dabei ist das Führungsmittel dazu eingerichtet und ausgebildet, dass mittels des Führungsmittels der Zylinder in eine bestimmungsgemäße Position innerhalb des Penis gedrückt werden kann (nachdem der Zylinder bereits in den Penis eingebracht wurde) und dass das Führungsmittel nach der bestimmungsgemäßen Positionierung von dem Zylinder entfernbar ist.

Ein erfindungsgemäßer Zylinder muss somit nicht durch Zug an einem extern gelegenen, d. h., aus dem Penis herausgeführten, Zylinderfaden bestimmungsgemäß positioniert werden, sondern kann über das entfernbare Führungsmittel durch Aufbringen eines (manuellen) Druckes auf den bereits in den Penis eingebrachten Zylinder in die bestimmungsgemäß vorgesehene Position überführt werden.

Im Zuge einer kompakten Ausgestaltung ist ferner erfindungsgemäß vorgesehen, dass das Führungsmittel zumindest mit einem Abschnitt in einer (äußeren) Zylinderwandung des Zylinders aufgenommen ist, die einen mit Fluid befüllbaren Zylinderinnenraum des Zylinders gegenüber einem den Zylinder umgebenden Außenraum begrenzt. Bei der äußeren Zylinderwandung handelt es sich folglich um eine den Zylinderinnenraum nach Art einer Mantelfläche bezogen auf eine Längsachse des Zylinders radial nach außen umgebende Wandung.

Gegebenenfalls handelt es sich hier um eine - im Vergleich zu Zylindern ohne ein (vergleichbares) Führungsmittel - verstärkte Zylinderwandung, so dass ein Führungsmittel sicher formschlüssig innerhalb der Zylinderwandung geführt und gelagert ist. So garantiert eine verstärkte Zylinderwandung, das beim Einführen und Entfernen des Führungsmittels keine Beschädigungen des Zylinderinnenraums auftreten kann, also das Führungsmittel beispielsweise unbeabsichtigt eine Innenwand des Zylinderinnenraums durchstößt, wodurch Fluid aus dem Zylinder austreten könnte.

Der Zylinder der Penisprothese kann dabei auf unterschiedliche Weise ausgebildet sein. So ist beispielsweise ein Zylinder denkbar, der sich durch die Befüllung mit dem Fluid ausdehnen und verlängern kann. Gleichfalls ist ein Zylinder denkbar, der zunächst in einem unbefüllten (leeren), schlaffen Zustand vorliegt und durch die Befüllung mit dem Fluid bei gleichbleibender Länge starr und fest wird, sich also bei (zusätzlicher) Befüllung mit dem Fluid nicht ausdehnt.

Andere Ausführungen können einen gegenüber dem distalen Ende des Zylinders härteren flexiblen Kern aufweisen, der bestimmungsgemäß beispielsweise in der Nähe des Beckens des Patienten implantiert wird und um den eine elastische Hülle gestülpt ist, die durch Druckbeaufschlagung expandiert werden kann.

Eine weitere alternative Ausführungsform weist einen gefalteten Mittelabschnitt auf, der an einen Blasebalg erinnert. Wird die Flüssigkeit in diesen Abschnitt geleitet, entfaltet und dehnt sich der Abschnitt entlang einer Längserstreckungsrichtung aus. Erfindungsgemäß ist das lösbar mit dem implantierbaren Zylinder verbundene Führungsmittel zumindest mit einem Abschnitt in einer dafür vorgesehenen Aufnahme des Zylinders aufgenommen. Das Führungsmittel ist hierbei in einer Faltung entlang einer Längsersteckungsrichtung des Zylinders in der äußeren Zylinderwandung geführt.

Beispielsweise ist die Aufnahme als längserstreckter Führungskanal oder als Bohrung in dem Zylinder ausgebildet, in den bzw. die ein stabförmiges Führungsmittel eingesteckt ist, obwohl dies nicht unter die Erfindung fällt. Gemäss der Erfindung ist vorgesehen eine Aufnahme für ein Führungsmittel in der äußeren Zylinderwandung dadurch auszubilden, dass die Zylinderwandung eine in Richtung auf den Zylinderinnenraum weisende Faltung aufweist. Über die nach (radial) nach innen weisende Faltung bzw. Einstülpung ist somit die Aufnahme für das Führungsmittel ausgeformt. Diese Faltung ist dabei vorzugsweise längserstreckt ausgebildet, so dass sie in Längserstreckungsrichtung des Zylinders an dessen Mantelfläche verläuft.

Alternativ oder ergänzend kann die Zylinderwandung flexibel sein. Dies hat sich insbesondere als vorteilhaft herausgestellt, wenn die flexible Zylinderwandung - wie erfindungsgemäß weiterhin vorgesehen - mindestens eine in Richtung auf den Zylinderinnenraum weisende Faltung (Einstülpung) aufweist. Auf diese Weise kann sich das Volumen des Zylinderinnenraums bei einer Befüllung mit Fluid durch Auffalten der Zylinderwandung vergrößern. Der Zylinder kann somit mit einer kompakteren, teilweise eingefalteten Zylinderwandung in den Penis eingeführt werden. Erst durch das (erstmalige) Befüllen des Zylinders mit einem Fluid, wie z.B. einer Kochsalzlösung, entfaltet sich die flexible Zylinderwandung, so dass sich der Zylinderinnenraum vergrößert.

In einer Weiterbildung weist der Zylinder eine Zylinderhülle auf, die die flexible Zylinderwandung zumindest teilweise umschließt und das Auffalten der flexiblen Zylinderwandung begrenzt. Über die (starre) Zylinderhülle wird somit die maximal entfaltete Form der Zylinderwandung vorgegeben und ein weiteres Auffalten der flexiblen Zylinderwandung auch bei ansteigendem Druck innerhalb des Zylinderinnenraums verhindert.

In einer Ausführungsvariante überdeckt die Zylinderhülle zumindest teilweise gerade (auch) die durch eine Faltung bzw. Einstülpung gebildete Aufnahme für das Führungsmittel. Auf diese Weise wird durch die Zylinderhülle während der Implantation des Zylinders in einen Penis eines Patienten zunächst eine Abschirmung des in der Faltung der Zylinderwandung geführten Führungsmittels bereitgestellt. Die Aufnahme in der Zylinderwandung bildet somit einen gegenüber der Zylinderhülle zurückgesetzten und von der Zylinderhülle überdeckten Bereich aus, in dem das Führungsmittel (gleitend) geführt ist. Vorzugsweise ist die Aufnahme hier längserstreckt und damit kanalförmig (als Führungskanal) ausgebildet.

Nach der bestimmungsgemäßen Positionierung des Zylinders kann das Führungsmittel aus der Aufnahme entfernt werden, insbesondere über eine Öffnung an der Zylinderhülle. Durch das Befüllen des Zylinders mit Fluid ist die Zylinderwandung entfaltbar, so dass die Aufnahme aus der Zylinderwandung herausgedrückt wird. Die Aufnahme steht damit insbesondere temporär bei unbefülltem Zylinder für die Positionierung des Zylinders zur Verfügung und wird durch ein späteres Befüllen des Zylinders zur Erzeugung einer Erektion des Penis beseitigt. Die zuvor mindestens in einem Bereich nach innen gefaltete, flexible Zylinderwandung ist hierbei bevorzugt durch das Befüllen des Zylinders mit Fluid (flach und/oder radial umlaufend) gegen die Innenseite der (geschlossenen) Zylinderhülle drückbar.

Die durch eine Faltung (Einstülpung) der Zylinderwandung bereitgestellte Aufnahme mündet in einer bevorzugten Ausführungsvariante in einen Kanal innerhalb des distalen Endes des Zylinders, so dass sich ein eingeführtes Führungsmitteln durch die (längserstreckte) Aufnahme an der Zylinderwandung entlang bis in diesen Kanal hinein und damit bis zu einem Zylinderende erstrecken kann.

Wie bereits eingangs kurz dargelegt, ist ein erfindungsgemäßer Zylinder stets ein Teil einer mehrteiligen Penisprothese, die wenigstens noch ein Reservoir zur Bereitstellung des Fluids umfasst, mit dem der Zylinder zur Erzeugung der Erektion (zusätzlich) befüllbar ist. Es ist dabei bekannt, durch Kompression des Reservoirs oder mittels eines unter Druck stehenden Reservoirs, den Zylinder (zusätzlich) zu befüllen. Darüber hinaus kann aber gleichfalls auch eine Pumpeinrichtung mit Steuerventilen als weitere Komponente der Penisprothese vorgesehen sein, wobei die Pumpeinrichtung und/oder das Reservoir auch in einen Zylinder integriert sein können.

Ein Reservoir einer Penisprothese befindet sich üblicherweise im Skrotum oder im Unterleib des Patienten. Insbesondere bei im Unterleib positioniertem Reservoir befindet sich die Pumpeinrichtung vorzugsweise im Skrotum, um die Bedienung der Penisprothese durch den Patienten zu erleichtern.

Bei allen genannten Ausführungsvarianten bleibt jedoch entscheidend, dass der mit Fluid befüllbare, d. h. insbesondere aufpump- oder aufblasbare, Zylinder der Penisprothese im Rahmen eines operativen Eingriffs bestimmungsgemäß in den Penis eingesetzt bzw. eingebracht und letztlich zur Gewährleistung einer einwandfreien Funktion genau positioniert werden muss. Mit dem erfindungsgemäßen Zylinder, der bereits vor seiner Einbringung in den Penis einen daran oder darin angebrachtes und lösbares Führungsmittel aufweist, über das der Zylinder in eine bestimmungsgemäße Position gedrückt werden kann und nicht mehr gezogen werden muss und das von dem Zylinder (vollständig) entfernbar ist, lässt sich nicht nur der operative Eingriff zur Implantierung des Zylinders bzw. der Penisprothese optimieren. Vielmehr muss auch die Glans penis bzw. die Eichel des Patienten nicht mehr durchstochen werden, wodurch der Eingriff für den Patienten weniger unangenehm ist.

Durch die erfindungsgemäße Ausbildung des Zylinders mit einem (ausreichend starren) Führungsmittel, über das der Zylinder in eine bestimmungsgemäße Position gedrückt werden kann, kann das Führungsmittel somit auch entlang einer Ausbringungsrichtung von dem Zylinder entfernbar sein, die zu einer Einbringungsrichtung unterschiedlich ist, entlang der Zylinder in den Penis des Patienten bestimmungsgemäß einzusetzen ist. So wird ein Zylinder der Penisprothese üblicherweise im Wesentlichen entlang einer Penislängsachse in einer Einbringungsrichtung, die zu der Glans penis weist, in den Penis eingesetzt. Während dann ein bisher in der Praxis üblicher Zylinder über einen an dem Zylinder befestigten Faden in die Einbringungsrichtung gezogen und der Faden von außen entfernt wird, zeichnet sich ein erfindungsgemäßer Zylinder vorzugsweise durch ein Führungsmittel aus, das in eine zu der Einbringungsrichtung unterschiedliche Ausbringungsrichtung von dem Zylinder vollständig entfernbar ist. Besonders vorteilhaft erscheint es in diesem Zusammenhang, wenn die Ausbringungsrichtung des Führungsmittels zu der Einbringungsrichtung und damit zu der Richtung, in die der Zylinder auch über das Führungsmittel gedrückt wird, entgegengesetzt ist. Auf diese Weise können über eine gemeinsame chirurgische Öffnung sowohl der Zylinder in den Penis eingebracht als auch das Führungsmittel von dem letztlich bestimmungsgemäß positionierten Zylinder entfernt werden.

Vorzugsweise ist das Führungsmittel zumindest mit einem Abschnitt in einem in Längserstreckungsrichtung des Zylinders liegenden Endbereich des Zylinders aufgenommen, wobei der implantierte Zylinder bei bestimmungsgemäßer Positionierung innerhalb des Penis über eine Außenseite dieses Endbereichs am Gewebe des Penis anliegt. Über eine derartige Anordnung des Führungsmittels an oder in dem Zylinder kann somit sichergestellt werden, dass über das Führungsmittel genau an den Stellen Druck zur Positionierung des Zylinders in den Zylinder eingeleitet wird, an denen der Zylinder auch (flächig) am Gewebe des Penis anliegen soll.

So ist dann auch in einer Weiterbildung vorgesehen, dass das Führungsmittel zumindest mit einem Abschnitt in einem Endbereich des Zylinders aufgenommen ist, der bei bestimmungsgemäßer Positionierung des Zylinders an einem der Glans penis zugewandten Ende innerhalb des Corpus cavernosum penis am Gewebe des Penis anliegt. Es wird mit anderen Worten ein Abschnitt des Führungsmittels an dem Ende des Zylinders angeordnet, das bei bestimmungsgemäß implantiertem und entlang der Penislängsachse erstreckendem Zylinder der Eichel zugewandt ist bzw. benachbart zu dieser liegt. So muss der aufpump- oder aufblasbare Zylinder gerade mit diesem Ende bestimmungsgemäß am Penisgewebe anliegen, damit eine dauerhaft beschwerdefreie und sichere Funktion der Penisprothese gewährleistet ist.

Erfindungsgemäß weist das Führungsmittel zumindest einen in dem Zylinder aufgenommenen oder an dem Zylinder angebrachten Abschnitt auf, der sich im Wesentlichen quer zu einer Längserstreckungsrichtung des Zylinders erstreckt. Da der Zylinder gerade entlang einer Längserstreckungsrichtung bzw. entlang einer Längserstreckungsachse in eine bestimmungsgemäße Position innerhalb des Penis über das Führungsmittel gedrückt werden soll, kann durch einen hierzu quer verlaufenden Abschnitt des Führungsmittels eine Krafteinleitung und -übertragung in Längserstreckungsrichtung verbessert werden.

Wird zum Beispiel ein stabförmiges Führungsmittel verwendet, das sich nur an oder in dem Zylinder entlang dessen Längserstreckungsrichtung erstreckt, könnte ohne quer verlaufenden Abschnitt gegebenenfalls nur punktuell Druck auf den Zylinder ausgeübt werden, um diesen hierdurch in Längserstreckungsrichtung zu bewegen. Über einen quer zur Längserstreckungsrichtung verlaufenden und insbesondere in dem Zylinder bzw. dessen Zylinderwandung aufgenommenen Abschnitt kann demgegenüber ein Linien- oder Flächenkontakt des Führungsmittelabschnitts hergestellt werden. An diesem Abschnitt kann somit eine Druckkraft effektiver in den Zylinder eingeleitet werden, um insbesondere sicherzustellen, dass der Zylinder mit einem distalen Ende (flächig) an dem Penisgewebe anliegt.

Bevorzugt erstreckt sich der im Wesentlichen quer verlaufende Abschnitt des Führungsmittels mit einer Länge quer zur Längserstreckungsrichtung des Zylinders, die in derselben Größenordnung liegt, wie eine sich im Bereich des Abschnitts ergebende Breite des Zylinders senkrecht zu seiner Längserstreckungsrichtung, und/oder mit einer Länge, die wenigstens halb so groß ist wie diese Breite. Bei einem Zylinder mit einer in Längserstreckungsrichtung des Zylinders verlaufenden Symmetrie- oder Mittelebene, zu der der Zylinder (bezüglich seiner äußeren Gestalt) spiegelsymmetrisch ist, bedeutet dies beispielsweise, dass ein quer verlaufender Abschnitt des Führungsmittels diese imaginäre Symmetrie- oder Mittelebene kreuzt, sofern sich der quer verlaufende Abschnitt entlang des Umfangs des Zylinders oder in den Zylinder hinein erstreckt.

So ist in einem konkreten Ausführungsbeispiel vorgesehen, dass sich ein stabförmiges Führungsmittel zumindest mit einem Abschnitt entlang der Längserstreckungsrichtung eines Zylinders mit kreisförmiger Grundfläche (Kreiszylinder) erstreckt und in einen quer zur Längserstreckungsrichtung verlaufenden Abschnitt an einem konusförmigen Ende des Zylinders mündet, wobei sich der quer verlaufende, das heißt hier, gebogene oder abgewinkelte Abschnitt des stabförmigen Führungsmittels über einen Mittelpunkt der Spitze des konusförmigen Endes hinweg erstreckt. Hierdurch kann eine auf das Führungsmittel in Längserstreckungsrichtung des Zylinders aufgebrachte Druckkraft über den quer verlaufenden (umgebogenen/abgewinkelten) Abschnitt besser in ein Ende bzw. eine Spitze des Zylinders eingeleitet werden, um eine Anlage der Oberfläche dieses Endes bzw. dieser Spitze am Penisgewebe sicherstellen zu können.

Anders ausgedrückt, ist das Führungsmittel vorzugsweise derart ausgebildet und in oder an dem Zylinder angeordnet, dass sich das Führungsmittel größtenteils axial (das heißt entlang einer Längsachse bzw. in Längsrichtung des Zylinders) erstreckt und das Führungsmittel zumindest an einem Ende des Zylinders, das bei einer bestimmungsgemäßen Implantierung ein distales Ende des Zylinders darstellt, nahezu radial ins Innere des Zylinders über eine Symmetrielinie bzw. -ebene des Zylinders hinweg verläuft.

Für eine Platz sparende Unterbringung des Führungsmittels und die gezielte Weitergabe der in das Führungsmittel eingeleiteten Druckkraft an den Zylinder kann ferner vorgesehen sein, dass sich das Führungsmittel im Inneren des Zylinders zumindest mit einem Abschnitt zwischen einem in Längserstreckungsrichtung des Zylinders liegenden Ende eines mit Fluid befüllbaren Zylinderinnenraums und einem in dieser Längserstreckungsrichtung liegenden Ende des Zylinders erstreckt. Ein solcher, vorzugsweise quer zur Längserstreckungsrichtung verlaufender Abschnitt des Führungsmittels befindet sich somit in einer Ausführungsform in einem distalen, der Glans penis zugewandten Endbereich des Zylinders innerhalb einer Zylinderwandung, die das Ende des mit Fluid befüllbaren Zylinderinnenraums von der Außenseite eines sich verjüngenden Zylinderendes bzw. der Außenseite einer Zylinderspitze trennt.

So soll sich der mit Fluid befüllbare Zylinderinnenraum für eine vollständige Erektion des Penis soweit wie möglich in eine aus anatomischen Gründen kegelförmig ausgebildete Spitze des Zylinders hinein erstrecken. Über die Anordnung eines Abschnitts des Führungsmittels genau in diese Spitze hinein (zwischen Zylinderinnenraum und der Außenseite der Spitze) kann auch die Spitze ohne weiteres über das Führungsmittel in die bestimmungsgemäße Position innerhalb des Penis hinein gedrückt werden.

Vorzugsweise ist das Führungsmittel zumindest teilweise aus einem elastischen oder flexibel verformbaren Material hergestellt, so dass es leicht manuell in eine in dem Zylinder vorgesehene, und zum Beispiel in Form eines Führungskanals ausgebildete Aufnahme einführ- oder einsteckbar ist, auch wenn die Aufnahme nicht gerade verläuft, sondern das Führungsmittel darin umgelenkt ist. So ist das Führungsmittel beispielsweise als ein biegsamer und/oder bereits abschnittsweise gebogener Führungsstab oder -draht aus einem biokompatiblen Material mit geeigneten physikalischen Eigenschaften, beispielsweise aus einem mit einem biokompatiblen Material wie Silikon beschichteten Federstahl, mit einem Durchmesser des Stabes bzw. Drahtes von ca. 1-2 mm ausgebildet.

Aufgrund des Einsatzes innerhalb des menschlichen Körpers wird das Führungsmittel ferner bevorzugt aus einem biokompatiblen Material hergestellt und/oder ist mit einem solchen biokompatiblen Material beschichtet.

Zum leichteren Einführen des Führungsmittels in eine Aufnahme des Zylinders und zum leichteren Entfernen des Führungsmittels aus einer Aufnahme des Zylinders kann vorgesehen sein, dass das Führungsmittel mit einem Gleitmaterial beschichtet oder aus einem Gleitmaterial hergestellt ist, das im Zusammenwirken mit dem Material, aus dem der Zylinder hergestellt ist, einen geringen Reibungskoeffizienten aufweist, so dass das Führungsmittel manuell von dem bestimmungsgemäß in dem Penis positionierten Zylinder entfernbar ist, ohne dass der Zylinder durch das Entfernen des Führungsmittels (merklich) verlagert wird. In diesem Zusammenhang sind verschiedene Materialkombinationen vorzugsweise mit wenigstens einem Kunststoffmaterial, wie Polyurethan oder Silikon, denkbar.

Alternativ oder ergänzend kann das Führungsmittel zumindest mit einem Abschnitt in einer Aufnahme des Zylinders angeordnet sein, die mit einem Gleitmittel gefüllt und/oder bei bereits darin angeordnetem Führungsmittel mit einem Gleitmittel (nachträglich) befüllbar ist, wobei das Gleitmittel die Reibung zwischen dem Führungsmittel und der Aufnahme reduziert. Ein solches Gleitmittel kann somit insbesondere (zäh-)flüssig, gelartig oder pastös sein.

Vorzugsweise ist die Aufnahme, zum Beispiel in Form eines Führungskanals, bereits vor dem ersten Einstecken bzw. Einführen des Führungsmittels zumindest teilweise mit einem schmierenden Gleitmittel gefüllt, so dass nach dem Implantieren des Zylinders das Führungsmittel auf einem durch das Gleitmittel gebildeten Gleitfilm wieder leichtgängig aus der Aufnahme des Zylinders herausgezogen werden kann.

Alternativ kann vorgesehen sein, dass zunächst das Führungsmittel in die Aufnahme eingesteckt oder eingeschoben wird und erst im Anschluss Gleitmittel in die Aufnahme zugegeben wird. In beiden Fällen weist die Aufnahme gegenüber den äußeren Abmessungen der aufzunehmenden Abschnitte des Führungsmittels größere Abmessungen auf, so dass das Führungsmittel (ohne Gleitmittel) unter Ausbildung einer Spielpassung zumindest abschnittsweise in der Aufnahme untergebracht bzw. unterbringbar ist.

Alternativ oder ergänzend kann gleichfalls vorteilhaft sein, das Führungsmittel vor seiner (erstmaligen) Einführung in die Aufnahme des Zylinders mit einem entsprechenden Gleitmittel zu versehen bzw. ein Gleitmittel auf die Außenflächen der in die Aufnahme einzuführenden Abschnitte des Führungsmittels aufzutragen.

Bevorzugt sind ein Gleitmaterial und ein Gleitmittel ebenfalls aus biokompatiblem Material.

Zur einfacheren Handhabung des Führungsmittels weist dieses in einer Ausführungsform einen Griffbereich auf, über den das Führungsmittel nach einer bestimmungsgemäßen Positionierung des Zylinders innerhalb des Penis von dem Zylinder entfernbar ist. Beispielhaft kann es sich hierbei um eine Öse, ein Querstück, einen profilierten und/oder gebogenen Abschnitt handeln. So kann über einen derartigen Griffbereich das Führungsmittel durch einen Operateur manuell insbesondere aus einer dafür vorgesehenen Öffnung an dem Zylinder aus dem Inneren des Zylinders, also zum Beispiel aus einer entsprechenden Aufnahme in der Zylinderwandung, herausgezogen werden.

Damit ein solcher Griffbereich leicht zugänglich ist, steht er vorzugsweise bezüglich der Längserstreckungsrichtung des Zylinders bzw. bezüglich dessen Längserstreckungsachse winklig von dem Zylinder hervor.

Um den Zylinder mit einem solchen (abstehenden) Griffbereich dennoch Platz sparend und somit mit geringer Packungsgröße anliefern zu können und gleichzeitig ein versehentliches Herausziehen des Führungsmittels zu vermeiden, kann es vorteilhaft sein, dass der Griffbereich des Führungsmittels in einer Anlieferungsposition des Zylinders zunächst an dem Zylinder gehalten und vor der Implantierung des Zylinders in einem Penis von dem Zylinder lösbar ist, so dass erst über den gelösten Griffbereich das Führungsmittel nach der bestimmungsgemäßen Positionierung des Zylinders in dem Penis vollständig von dem Zylinder entfernbar ist. Derart ist also der Griffbereich zunächst in einer Anlieferungsposition an dem Zylinder lösbar fixiert, zum Beispiel über eine punktuelle Klebung oder einen schmalen (gegebenenfalls perforierten) Streifen aus angespritztem Kunststoffmaterial, die bzw. der bei einem gezielten Zug an dem Griffbereich versagt und so den Griffbereich freigibt.

Neben einem Zylinder für eine Penisprothese betrifft die vorliegende Erfindung auch eine Penisprothese mit wenigstens einem Zylinder entsprechend den vorangegangenen Ausführungsbeispielen, der in einen Penis eines Patienten implantierbar, zur Erzeugung einer Erektion des Penis mit Fluid befüllbar und darüber hinaus mit einem entsprechenden Führungsmittel versehen ist.

Wie bereits zuvor erläutert, ist ein wesentlicher Vorteil einer erfindungsgemäßen Ausführungsform eines Zylinders für eine Penisprothese, dass das Führungsmittel insbesondere entlang einer Ausbringungsrichtung von dem in dem Penis bestimmungsgemäß positionierten Zylinder entfernbar ist, die entgegengesetzt zu einer Einbringungsrichtung ist, entlang derer der Zylinder üblicherweise in den Penis einzubringen und in die der Zylinder über das Führungsmittel in ein bestimmungsgemäße Position innerhalb des Penis zudrücken ist. Dementsprechend kann ein Operateur bei der operativen Implantierung eines erfindungsgemäßen mit Fluid befüllbaren Zylinders einer Penisprothese entsprechend den nachfolgenden Schritten vorgehen:
- Einbringen des Zylinders mit daran angebrachtem Führungsmittel im Wesentlichen entlang einer Einbringungsrichtung in den Penis,
- Drücken des in dem Penis liegenden Zylinders mittels des Führungsmittels im Wesentlichen entlang der Einbringungsrichtung, bis der Zylinder eine bestimmungsgemäße (End-)Position innerhalb des Penis einnimmt,
- Entfernen des Führungsmittels durch Ab- oder Herausziehen von dem in dem Penis bestimmungsgemäß positionierten Zylinder entlang einer Ausbringungsrichtung, die im Wesentlichen entgegengesetzt zu der Einbringungsrichtung ist.
Im folgenden beschrieben ist auch ein Verfahren zur Implantierung eines erfindungsgemäßen mit Fluid befüllbaren Zylinders einer Penisprothese mit diesen Schritten.

Wie bereits zuvor ausführlich dargelegt, ist somit der Zylinder in eine bestimmungsgemäße Position, in der z.B. ein Zylinderende an seiner Außenseite flächig an dem der Glans penis zugewandten Ende des Corpus cavernosum penis anliegt, drückbar, während in der Praxis bisher übliche Zylinder und Verfahren ein Ziehen des Zylinders über einen an dem Zylinder befestigen und durch die Glans penis hindurch zuführenden Faden vorsehen.

Weiterhin ist das Führungsmittel entgegen der ursprünglichen Einbringungsrichtung und damit der entgegen der Richtung, in die der Zylinder innerhalb des Penis üblicherweise zu drücken ist, aus dem Zylinder entfernbar, so dass das Führungsmittel vorzugsweise aus der chirurgisch geschaffenen Öffnung im Hautgewerbe des Patienten durch den Operateur herausnehmbar ist, über die bereits zuvor die Einbringung des Zylinders zusammen mit dem Führungsmittel erfolgte. Zusätzlich zu der Vermeidung einer Verletzung der Glans penis bzw. der Eichel des Patienten durch das Durchstechen mit einer Nadel kann somit beim Einsatz des erfindungsgemäßen Zylinders für eine Penisprothese der chirurgische Aufwand für die Implantierung der Penisprothese erheblich verringert werden.

Mögliche Ausführungsbeispiele sind auch durch die Unteransprüche angegeben. Weitere Vorteile und Merkmale der Erfindung werden bei der nachfolgenden Beschreibung eines Ausführungsbeispiels anhand der Figuren deutlich werden.

Es zeigen:
- Fig.1: eine Penisprothese aus dem Stand der Technik mit einem Reservoir, einer Pumpeinrichtung und zwei in einen Penis implantierten und mit Fluid befüllbaren Zylindern;
- Fig. 2 - 6: ein Ausführungsbeispiel eines Zylinders in verschiedenen Ansichten, welches nicht Gegenstand der Erfindung ist.;
- Fig. 7-9C: ein Ausführungsbeispiel eines erfindungsgemäßen Zylinders in verschiedenen Ansichten.

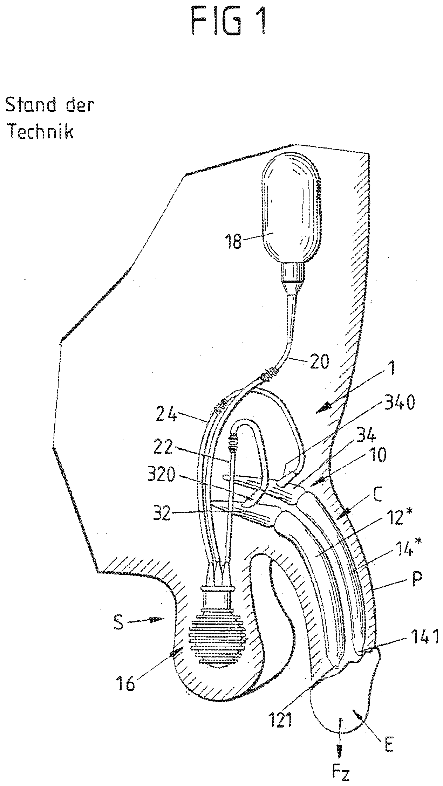

In der Figur 1 ist zunächst eine aus dem Stand der Technik bekannte Penisprothese 1 wiedergegeben, wie sie in der US-A 4,566,446 dargestellt ist.

Eine solche Penisprothese 1 umfasst zwei parallel zueinander in einen Penis P zu implantierende Zylinder 12*, 14*, ein Fluid bereitstellendes Reservoir 18 sowie eine Pumpeinrichtung 16, über die Fluid des Reservoirs 18 durch Leitungen 20, 22, 24 von den und in die Zylinder 12*, 14* gefördert werden kann, um eine Erektion des Penis P zu steuern.

Die Penisprothese 1 ist vollständig im Körper des Patienten implantiert, wobei das Reservoir 18 im Unterleib des Patienten, die Pumpeinrichtung 16 im Skrotum S des Patienten und die längserstreckten, flexiblen Zylinder 12*, 14* im Corpus cavernosum penis C des Penis P des Patienten untergebracht werden.

Die Zylinder 12*, 14* weisen an ihren im implantierten Zustand in Richtung des Beckens weisenden Enden jeweils einen Anschlussbereich 10 auf, in dem separate Anschlussstücke 32, 34 mit je einem Zylinder 12*, 14* verbunden und vorzugsweise auf oder in den jeweiligen Zylinder 12*, 14* gesteckt sind. Diese Anschlussstücke 32, 34 sind üblicherweise aus einem im Vergleich zu dem Material des übrigen Zylinders 12*, 14* härterem und festerem Material gefertigt, um eine sichere Abstützung des Zylinders 12* bzw. 14* am Beckenknochen des Patienten gewährleisten zu können. Darüber hinaus stellen die Anschlussstücke 32, 34 jeweils einen Anschluss 320 bzw. 340 bereit, an dem das Anschlussstück 32, 34 mit einer Leitung 22 bzw. 24 verbindbar ist, um eine fluidtechnische Kopplung des Zylinders 12*, 14* mit der in dem Skrotum S angeordneten Pumpeinrichtung 16 herzustellen.

Die Zylinder 12*, 14* erstrecken sich im implantierten Zustand innerhalb des Penis P vom Becken des Patienten hin zur Eichel bzw. Glans penis E, so dass sie bei bestimmungsgemäßer Positionierung mit einem sich verjüngenden Zylinderende 121, 141 jeweils unmittelbar an dem der Glans penis E zugewandten Ende des Corpus cavernosum penis C anliegen. So ist es für die Erzeugung einer Erektion des Penis P durch die mit (zusätzlichem) Fluid befüllbaren und sich dadurch versteifenden Zylinder 12*, 14* nötig, dass diese flächig am Penisgewebe anliegen und sich insbesondere im Bereich der Zylinderenden 121 bzw. 141 keine Hohlräume zwischen den Zylindern 12*, 14* und Penisgewebe ausbilden.

Hierfür weisen die im Wesentlichen kreiszylinderförmigen Zylinder 12*, 14* die sich konisch verjüngenden Zylinderenden 121 und 141 auf. Diese sind im Übrigen wie die ebenfalls konus- bzw. kegelförmigen Anschlussstücke 32, 34 an die menschliche Anatomie angepasst, um einen sicheren Sitz der Penisprothese 1 im Körper bzw. insbesondere der Zylinder 12*, 14* im Corpus cavernovum penis C und am Beckenknochen zu realisieren.

Um die Zylinder 12*, 14* nach einer ersten Einbringung in den Penis P letztlich bestimmungsgemäß innerhalb des Penis P zu positionieren, die Zylinder 12*, 14* also ausgehend von einer ersten Position innerhalb des Penis P, in der sie ein Operateur zunächst angeordnet hat, in eine bestimmungsgemäße (End-) Position zu verstellen bzw. zu bewegen, ist es bisher in der chirurgischen Praxis üblich, die Zylinder 12*, 14* über einen an ihren Zylinderenden 121 bzw. 141 angebrachten Zylinderfaden in Richtung der Glans penis E zu ziehen. Die Zylinder 12*, 14* werden also üblicherweise beckenseitig in den Penis P in Richtung der Glans penis E eingelegt oder eingeschoben und anschließend durch einen mit den Zylindern 12*, 14* jeweils verbundenen Faden, der über eine die Glans penis E von innen nach außen durchstechende Nadel rausgeführt ist, in Richtung der Glans penis E in eine bestimmungsgemäße Endposition gezogen. Dieses Vorgehen ist durch die in der Figur 1 veranschaulichte Zugkraft F_{Z} veranschaulicht.

Diese der simplen Ausgestaltung der Zylinder 12*, 14* geschuldete Methode ist somit nicht nur mit zusätzlichem operativen Aufwand, sondern auch mit einer zusätzlichen Verletzung der Glans penis E verbunden, die für den Patienten bis zur Genesung unangenehm und schmerzintensiv ist.

Die erfindungsgemäße Lösung sieht daher vor, einen Zylinder mit einem Führungsmittel bereitzustellen, mittels dem der Zylinder in eine bestimmungsgemäße Position innerhalb des Penis P gedrückt werden kann und dass nach der bestimmungsgemäßen Positionierung von dem Zylinder (vollständig) entfernbar ist.

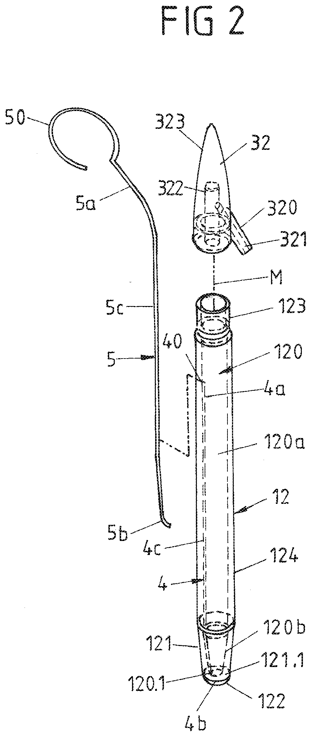

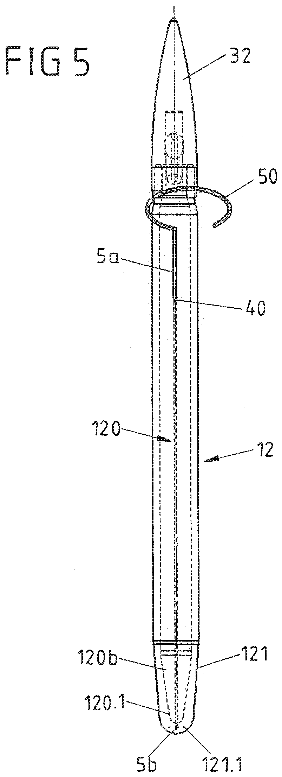

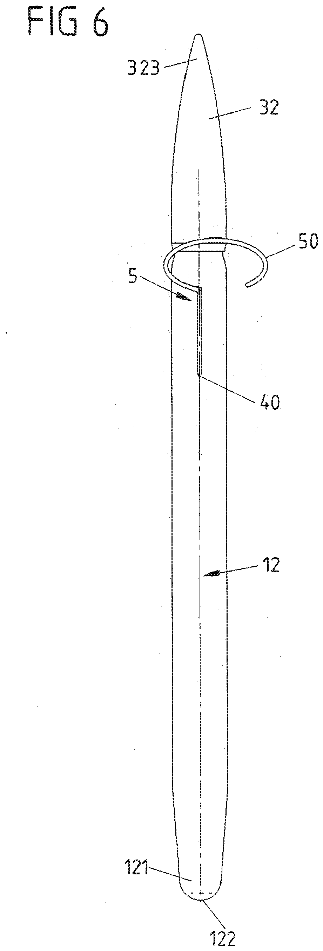

Die Figuren 2, 3A-3B, 4, 5 und 6 zeigen ein nicht erfindungsgemässes Ausführungsbeispiel eines solchen Zylinders 12, der insbesondere anstelle eines Zylinders 12* oder 14* der Penisprothese 1 der Figur 1 eingesetzt werden kann.

Der Zylinder 12 ist hier gleichfalls als langgestrecktes hülsenförmiges und Bauteil ausgebildet, das einen kreiszylinderförmigen Teilbereich 120a, der sich über den Großteil der Länge des Zylinders 12 erstreckt, und einen sich daran anschließenden (zweiten) Teilbereich 120b aufweist, der ein sich bis zu einer abgerundeten Zylinderspitze 121.1 hin verjüngendes Zylinderende 121 umfasst. Innerhalb des hohlen Zylinders 12 ist ein Zylinderinnenraum 120 vorgesehen, der mit Fluid befüllbar ist, um das Versteifen bzw. Erschlaffen des flexiblen Zylinders 12 und damit eine Erektion des Penis P bei implantiertem Zylinder 12 zu steuern.

Die Form des von einer dünnen Zylinderwandung 124 eingefassten Zylinderinnenraums 120 entspricht der äußeren Gestalt des Zylinders 120, so dass sich auch ein Ende des Zylinderinnenraums 120 in dem Teilbereich 120b zu der Zylinderspitze 121.1 hin konisch verjüngt. Dabei bildet das Zylinderende 121 mit der Zylinderspitze 121.1 das distale Ende des Zylinders 12, das bei bestimmungsgemäßer Implantierung des Zylinders 12 in den Penis P der Glans penis E zugewandt ist.

An dem gegenüberliegenden Ende umfasst der Zylinder 12 ein sich gleichfalls verjüngendes, kegelförmiges Anschlussstück 32, das auf einen Anschlussabschnitt 123 des Zylinders 12 als separate Komponente aufgesetzt ist. Das Anschlussstück 32 mit seiner Spitze 323 weist in seinem Inneren Leitungskanäle 321, 322 auf, über die Fluid von und zu einem Anschluss 320 des Anschlussstücks 32 sowie hierüber zu und von dem Zylinderinnenraum 120 geleitet werden kann.

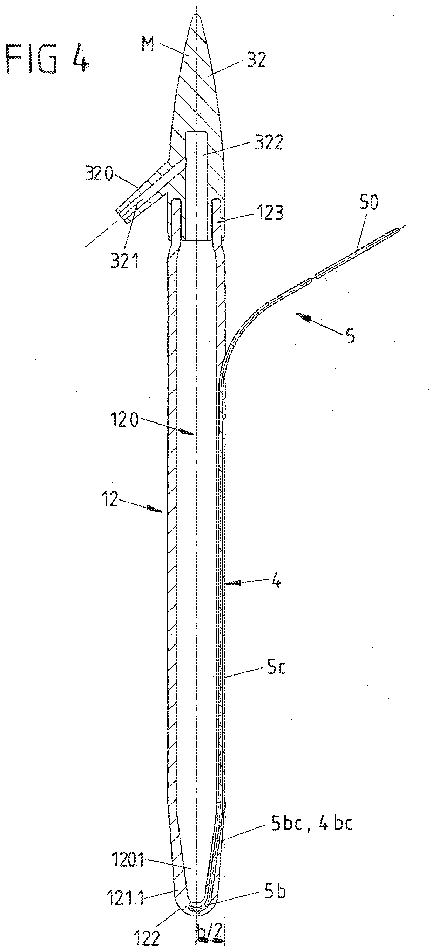

Wie insbesondere anhand der Schnittdarstellung der Figur 4 ersichtlich ist, ist das Anschlussstück 32 formschlüssig mit dem Anschlussabschnitt 123 verbunden, so dass ein kragenförmiger, hülsenartiger Bereich des Anschlussabschnitts 123 in eine Außenwandung des Anschlussstücks 32 eingreift, wenn das Anschlussstück 32 bestimmungsgemäß mit dem Rest des Zylinders 12 dichtend verbunden ist.

Der längserstreckte Zylinder 12 erstreckt sich somit entlang einer imaginären geraden Mittellinie M, auf der sowohl die Spitze 323 des Anschlussstücks 32 als auch die Zylinderspitze 121.1 liegen. Als Längserstreckungsrichtung des Zylinders 12 wird somit vorliegend die parallel zur Mittellinie M verlaufende Richtung angesehen, die von der Spitze 323 zu der Zylinderspitze 121.1 weist.

Um die bestimmungsgemäße Positionierung des Zylinders 12 innerhalb eines Penis P zu vereinfachen und den Zylinder 12 nicht in eine bestimmungsgemäße Position ziehen zu müssen, weist der Zylinder 12 ein lösbar mit dem Zylinder 12 verbundenes Führungsmittel 5 auf. Dieses Führungsmittel 5 wird vorliegend durch einen elastisch oder flexibel verformbaren Führungsstab aus biokompatiblem Material gebildet, der in einer als Führungskanal 4 ausgebildeten Aufnahme des Zylinders 12 teilweise aufgenommen ist und sich nur mit einem einen Griffbereich 50 aufweisenden Zuführabschnitt 5a außerhalb des Zylinders 12 erstreckt, wenn das Führungsmittel 5 vor der Implantierung des Zylinders 12 in den Penis P bestimmungsgemäß an den Zylinder 12 angeordnet ist.

Die Aufnahme bzw. der Führungskanal 4 für die formschlüssige Lagerung des Führungsmittels 5 in dem Zylinder 12 erstreckt sich innerhalb der Zylinderwandung 124, die den Zylinderinnenraum 120 begrenzt. Der Führungskanal 4 verläuft dabei ausgehend von einer Öffnung 40, über die das Einführen und Herausziehen des Führungsmittels 5 aus dem Zylinder 12 möglich ist, entlang dem kreiszylinderförmigen Teilbereich 120a über den sich verjüngenden Teilbereich 120b bis in die Zylinderspitze 121.1 innerhalb der Zylinderwandung 124 (vergleiche insbesondere Figuren 2, 3A und 4). Der Führungskanal 4 erstreckt sich somit ausgehend von der Öffnung 40 über einen Zuführabschnitt 4a und einen sich daran anschließenden Mittelabschnitt 4c zu einem in der Zylinderspitze 121.1 endenden Endabschnitt 4b axial über einen Großteil der Länge des Zylinders 12.

Um über das in den Führungskanal 4 eingesetzte Führungsmittel 5 einen ausreichenden Druck auf die Zylinderspitze 121.1 ausüben zu können, so dass der Zylinder 12 mit seinem Zylinderende 121 wie gewünscht in Anlage mit dem Penisgewebe kommt, ist der Endabschnitt 4b des Führungskanals 4 als zu der Längserstreckungsrichtung des Zylinders 12 quer verlaufender Abschnitt ausgebildet. Ein in den Führungskanal 4 eingebrachtes Führungsmittel 5 wird somit innerhalb des Führungskanals 4 entsprechend der Kontur des Zylinders 12 bzw. seines Zylinderinnenraums 120 umgelenkt und mündet als gebogener oder abgewinkelter Endabschnitt 5b in der Zylinderspitze 121.1 innerhalb der Zylinderwandung 124. Derart befindet sich der Endabschnitt 5b des Führungsmittels 5 an dem Zylinderende 121 zwischen einem Innenraumende 120.1 des Zylinderinnenraums 120 und der Außenseite der Zylinderspitze 121.1. Hierbei bildet die Zylinderspitz 121.1 an ihrer Außenseite eine Kontaktfläche 122 aus, die bei bestimmungsgemäßer Positionierung des Zylinders 12 an dem dem Glans penis E zugewandten Penisgewebe anliegt.

Um über das Drücken des Führungsmittels 5 in Richtung der Zylinderspitze 121.1 eine möglichst geradlinige Bewegung des gesamten Zylinders 12 in seine Längserstreckungsrichtung bewirken zu können, erstreckt sich in der Zylinderspitze 121.1 der Führungskanal 4 und damit der Endabschnitt 5b des Führungsmittels 5 über den Mittelpunkt der Zylinderspitze 121.1 hinweg in die Zylinderwandung 124 hinein. Dies ist vor allem gut aus der Schnittdarstellung aus Figur 4 ersichtlich. Der quer verlaufende Endabschnitt 5b des Führungsmittels 5 ist daher in dieser Ausführungsform etwa halb so lang wie eine Breite b des Zylinders 12 bzw. hier halb so lang wie ein Durchmesser des Zylinders 12. Derart kann die Zylinderspitze 121.1 genau platziert werden, indem über das Führungsmittel 5 der Zylinder 12 während der Implantation stabilisiert und mittels Drückens des Führungsmittels 5 eine bestimmungsgemäße Positionierung des Zylinders 12 direkt an der Operationsstelle ermöglicht wird.

Wie insbesondere anhand der Figur 2 ersichtlich ist, handelt es sich hier bei dem Führungsmittel 5 um einen Führungsstab bzw. einen Führungsdraht, der in den Führungskanal 4 eingeführt und dort vor der Implantierung des Zylinders 12 in den Penis P gelagert ist. Das Führungsmittel 5 ist wenigstens derart flexibel ausgebildet, dass es sich (manuell) in den Führungskanal 4 einführen lässt und entsprechend der Kontur des Zylinders 12 innerhalb der Zylinderwandung 124 umgelenkt werden kann. So schließt sich an den Mittelabschnitt 4c des Führungskanals 4 ein zu der Zylinderspitze 121.1 schräg verlaufender, durch die sich verjüngende Form des Zylinderendes 121 bedingter Übergangsabschnitt 4bc an, der in den im Wesentlichen quer zu dem Mittelabschnitt 4c verlaufenden Endabschnitt 4b mündet. Dementsprechend weist auch das formschlüssig in dem Führungskanal 4 aufgenommene Führungsmittel 5 einen Mittelabschnitt 5c, einen Übergangsabschnitt 5bc und einen Endabschnitt 5b mit entsprechendem Verlauf auf, wenn das Führungsmittel 5 in den Führungskanal eingeführt, also vorzugsweise eingeschoben wurde.

Der winklig von dem Zylinder 12 bzw. dessen Außenseite hervorstehende Griffbereich 50 ist in dem vorliegenden Ausführungsbeispiel durch eine geschlitzte Öse oder eine offene Schlinge gebildet. Der Durchmesser dieser Öse oder Schlinge ist dabei so groß gewählt, dass ein Finger durch sie hindurchgeführt und damit von einem Finger ergriffen werden kann, um das Führungsmittel 5 durch Herausziehen aus dem Führungskanal 4 von dem Zylinder 12 zu entfernen, nachdem der Zylinder 12 bestimmungsgemäß innerhalb des Penis P positioniert wurde.

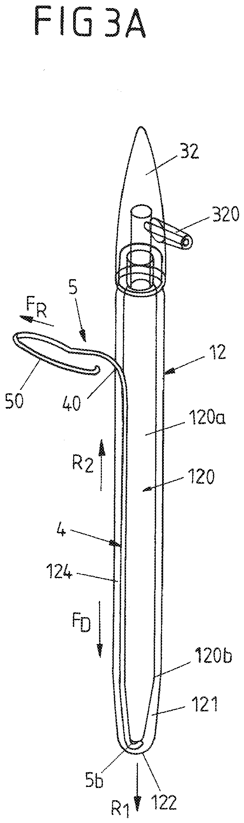

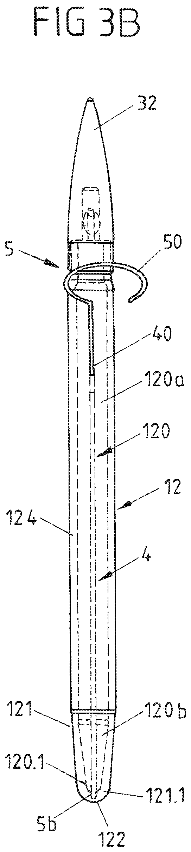

Der Griffbereich 50 des Führungsmittels 5 kann insbesondere derart flexibel ausgelegt sein, dass er in einer Anlieferungsposition des Zylinders 12 den Zylinder 12 radial umgreift, sich also der Zylinder 12 durch die Öffnung des Griffbereichs 50 hindurch erstreckt. Der Griffbereich 50 ist somit gegebenenfalls im Bereich des Anschlussabschnitts 123 an dem Zylinder 12 durch Einhängen festlegbar. Vor der Implantierung des Zylinders 12 kann der Griffbereich 50 dann von dem Zylinder 12 gelöst werden, so dass er sich entsprechend den Figuren 3A, 3B, 4, 5 und 6 winklig von dem Zylinder 12 weg erstreckt und damit von einem Operateur besser greifbar ist.

Zur Arretierung des Griffs bzw. Griffbereichs 50 kann beispielsweise auch ein zwischen dem aufgesetzten Anschlussstück 32 und dem übrigen Zylinder 12 im Bereich des Anschlussabschnitts 123 ausgebildete Absatz oder eine (dort) ausgebildete Rille oder Nut dienen.

Alternativ hierzu kann selbstverständlich auch vorgesehen sein, dass der Griffbereich in einer Anlieferungsposition anderweitig, z. B. über eine Klebestelle oder einen angespritzten dünnen Kunststoffstreifen, (manuell) lösbar an dem Zylinder 12 fixiert ist.

Mit der Figur 3A wird die Funktionsweise des gezeigten Ausführungsbeispiels sowie eines damit möglichen Vorgehens bei der Implantierung des Zylinders 12 nochmals näher veranschaulicht. So kann mit der erfindungsgemäßen Ausgestaltung eines Zylinders für eine Penisprothese dieser zunächst zusammen mit dem daran angeordneten Führungsmittel 5 entlang einer Einbringungsrichtung R₁ in den Penis P eingeführt werden, wobei diese Einbringungsrichtung R₁ im Wesentlichen von dem Becken des Patienten zu der Eichel bzw. der Glans penis E des Patienten weist. Durch Aufbringen einer Druckkraft F_{D} in Einbringungsrichtung R₁ über das Führungsmittel 5 ist der Zylinder 12 bestimmungsgemäß innerhalb des Penis P positionierbar und insbesondere mit seinem Zylinderende 121 bestimmungsgemäß innerhalb der Corpus cavernosum penis C in Anlage zu Penisgewebe bringbar. Nach Drücken des Zylinders 12 in eine bestimmungsgemäße Position mittels des Führungsmittels 5 ist das Führungsmittel 5 aus dem Zylinder 12 entgegengesetzt zu der ursprünglichen Einbringungsrichtung R₁ entlang einer Ausbringungsrichtung R₂ manuell herausziehbar, indem ein Operateur an dem Griffbereich 50 mit einer Ausziehkraft F_{R} entsprechend der Figur 3A zieht.

Damit dabei keine unerwünschte Verstellung des bereits bestimmungsgemäß positionierten Zylinders 12 auftritt, ist eine ausreichende Gleitfähigkeit des Führungsmittels 5 bzw. eine entsprechende Schmierung mit einem Gleitmittel innerhalb des Führungskanals 4 vorgesehen. So kann das Führungsmittel 5 aus einem Gleitmaterial hergestellt oder mit einem solchen beschichtet sein und/oder es ist ein flüssiges oder pastöses Gleitmittel innerhalb des Führungskanals 4 vorgesehen.

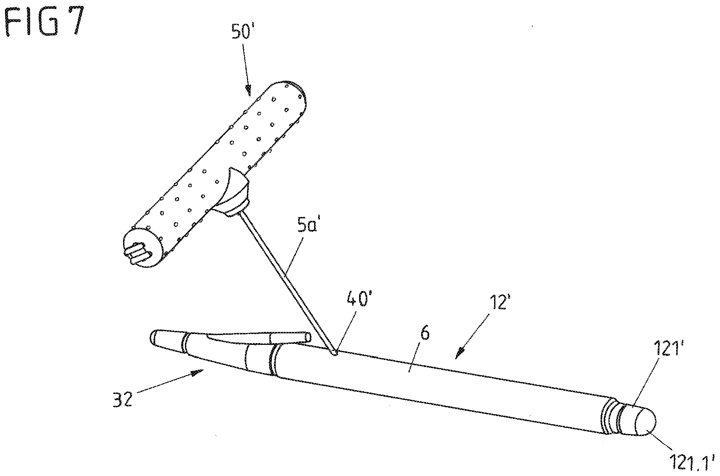

Die Figuren 7 bis 9C veranschaulichen eine mögliche Ausführungsvariante eines erfindungsgemäßen Zylinders 12' für eine Penisprothese, dessen Positionierung innerhalb eines Penis P durch ein entfernbares Führungsmittel 5' erleichtert ist. Hierbei sind wesensgleiche oder (funktional) übereinstimmende Komponenten mit identischen Bezugszeichen wie in den vorangegangenen Figuren 2 bis 6 gekennzeichnet und mit einem ergänzendem Apostroph (') versehen.

Die Figur 7 zeigt in einer perspektivischen Ansicht den einsetzbaren Zylinder 12' mit dem Anschlussstück 32 und einem aus einer Öffnung 40' in einer Zylinderhülle 6 hervorstehenden Führungsmittel 5', an dem ein gegenüber der Ausführungsform der vorangegangenen Figuren abgewandelter Griffbereich 50' ausgebildet ist. Dieser Griffbereich 50' ist mit einem Querriegel ausgebildet, der von einer Hand vollständig umfasst werden und damit das manuelle Herausziehen des Führungsmittels 5' aus dem Zylinder 12' erleichtert.

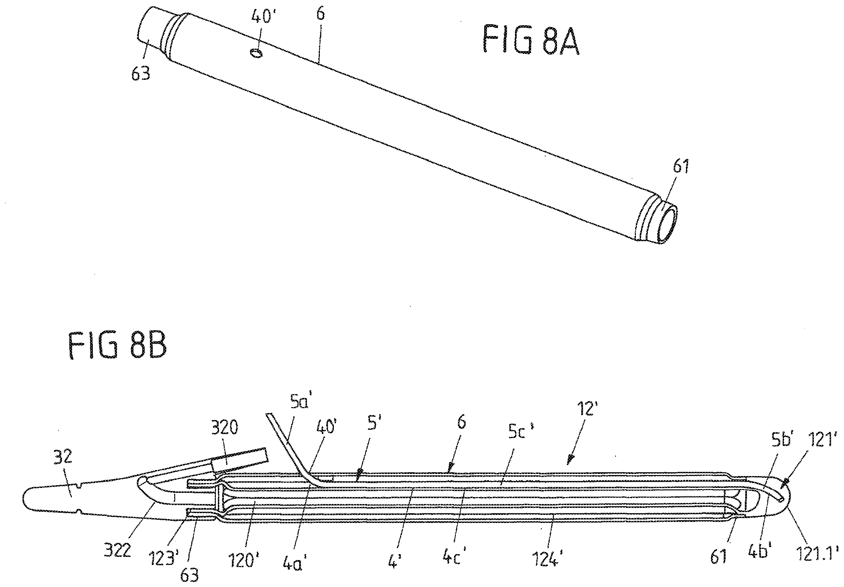

Die Figur 8A zeigt in perspektivischer Einzeldarstellung vergrößert die Zylinderhülle 6, die in der vorliegenden Ausführungsvariante eine den Zylinderinnenraum 120' des Zylinders 12' (in dem Teilbereich 120a') berandende (flexible) Zylinderwandung 124' umgibt. Die Zylinderhülle 6 ist im Wesentlichen hülsenförmig oder schlauchförmig ausgebildet. Ferner ist sie bevorzugt aus Silikon oder einem anderen biokompatiblen Material hergestellt. Die Zylinderhülle 6 ist hierbei zumindest so starr ausgeführt, dass sie die Ausdehnung eines innerhalb der Zylinderhülle 6 angeordneten Zylinderkerns, der durch eine einen Zylinderinnenraum 120' umgebende Zylinderwandung 124' definiert ist, auf ein gewünschtes Maß begrenzt.

Die Zylinderhülle 6 dient in dem gezeigten Ausführungsbeispiel nicht nur als Befestigung des Zylinderkerns an dem Anschlussstück 32, sondern begrenzt auch eine Ausdehnung des Zylinderkerns bzw. der Zylinderwandung 124' in radialer Richtung wie auch in Längsrichtung.

Die hülsenförmige Zylinderhülle 6 weist an ihrem einen Hüllenende 61 einen Absatz auf, an dem die Zylinderhülle 6 an einem Auflagebereich 126' des (massiven) Zylinderendes 121' an dem distalen Ende des Zylinders 12' (am Beginn des Teilbereichs 120b') anliegt.

An dem gegenüberliegenden Hüllenende weist die Zylinderhülle 6 einen Anschlussabschnitt 63 auf. Dieser Anschlussabschnitt 63 ist so dimensioniert, dass er einen Anschlussabschnitt 123' des Zylinderkerns umschließt und das Anschlussstück 32 auf ihn aufgesetzt werden kann. Das (bei bestimmungsgemäßer Positionierung des Zylinders 12' dem Becken eines Patienten zugewandte) Ende des Zylinders 12' mit dem Anschlussabschnitt 63 ist somit zusammen mit dem Ende des Zylinderkerns in dem Anschlussstück 32 fixiert. An dem Anschlussstück 63 der Zylinderhülle 6 kann insofern eine zylindrische Klebefläche vorgesehen sein, um das Anschlussstück 32 an der Zylinderhülle 6 zu befestigen.

In der Figur 8B ist der Zylinder 12' entsprechend der Figur 7 in einem Längsschnitt dargestellt. Insbesondere anhand der Figur 8B ist hierbei gut ersichtlich, dass der Zylinder 12' eine Aufnahme in Form eines längserstreckten Führungskanals 4' in der Zylinderwandung 124' aufweist. Dieser Führungskanal 4' erstreckt sich entlang der Längserstreckungsrichtung des Zylinders 12' über einen Großteil dessen Länge.

Die Aufnahme bzw. der Führungskanal 4' wird hier durch eine (Dehn-)Falte an der Zylinderwandung 124' bereitgestellt, die radial nach innen gerichtet ist. Mit anderen Worten weist die von der Zylinderhülle 6 eingefasste Zylinderwandung 124' in dem in der Figur 8B gezeigten Zustand (vor einem erstmaligen Befüllen mit einem Fluid) eine in den Zylinderinnenraum 120' weisende Faltung bzw. Einstülpung auf, in der das Führungsmittel 5' bis an die Zylinderspitze 121.1' heran innerhalb der Zylinderwandung 124' geführt ist. Über diesen hierdurch gegenüber der Zylinderhülle 6 zurückgesetzten Bereich der Zylinderwandung 124' wird somit der Führungskanal 4' bereitgestellt, der von der den Zylinderkern umgebenden Zylinderhülle 6 vollständig überdeckt ist.

Dieser Führungskanal 4' verläuft entlang der Längserstreckungsrichtung des Zylinders 12' und mündet in einen kanalförmigen Endabschnitt 4b' in dem sich verjüngenden Zylinderende 121' des Zylinders 12'. Dieser Endabschnitt bzw. Kanal 4b' weist - wie der Endabschnitt 4b des Ausführungsbeispiels der Figuren 2 bis 6 - einen gebogenen bzw. abgewinkelten Verlauf innerhalb des die Zylinderspitze 121.1' ausbildenden, massiven Zylinderendes 121' auf. Der gebogene Endabschnitt 4b' verläuft derart gebogen innerhalb des Zylinderendes 121', dass das Führungsmittel 5' mit seinem Endabschnitt 5b' hierin quer zu einer Längserstreckungsrichtung des Zylinders 12' schräg radial nach innen bis in die Spitze des Zylinderendes 121', also bis zumindest an die Mittellinie des Zylinders 12' heran, geführt ist.

Das an dem Zylinder 12' angeordnete Führungsmittel 5' erstreckt sich ausgehend von einem Zuführabschnitt 5a', an dem der Griffbereich 50' angeordnet ist, durch die Öffnung 40' an der Zylinderhülle 6 hindurch zu einem Zuführabschnitt 4a' des Führungskanals 4'. Dieser Zuführabschnitt 4a' geht in einen Mittelabschnitt 4c' über, bevor er in den Endabschnitt 4b' mündet. Während der Zuführabschnitt 4a' und der Mittelabschnitt 4c' hierbei also durch die Faltung der Zylinderwandung 124' ausgebildet sind, ist der Endabschnitt 4b' in dem massiven Zylinderende 121' ausgebildet.

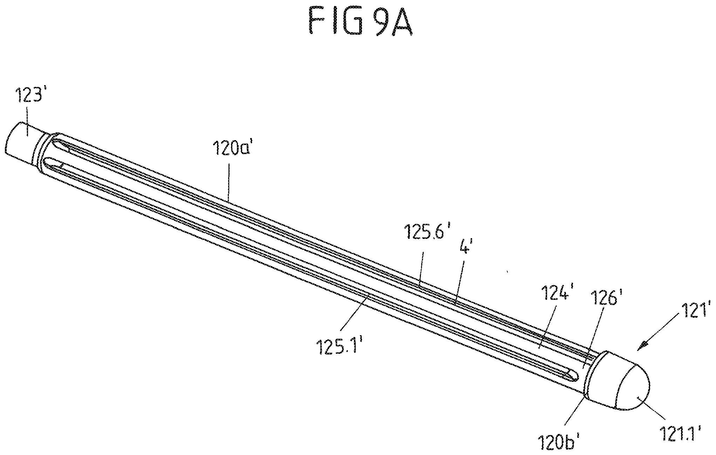

Eine den Führungskanal 4' (mit-)definierende (Dehn-)Falte 125.6', in der ein Mittelabschnitt 5c' des Führungsmittels 5' ruht, wenn über das Führungsmittel 5' der Zylinder 12 in eine bestimmungsgemäße Position gedrückt werden soll, ist in den weiteren Figuren 9A bis 9C in weiteren Detaildarstellungen ersichtlich.

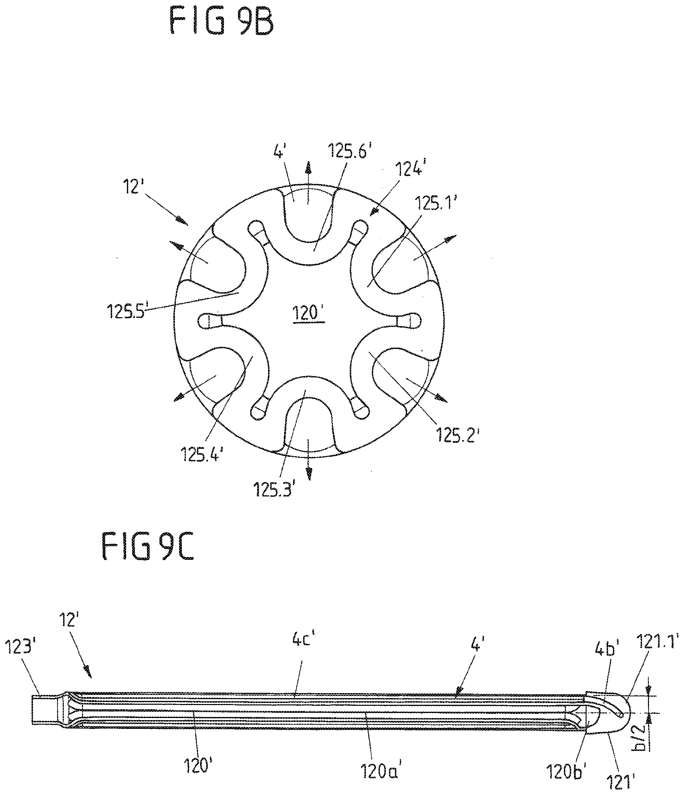

So weist die flexible Zylinderwandung 124', die den Zylinderkern des Zylinders 12' bildet und den Zylinderinnenraum 120' (dichtend) umschließt, mehrere (hier insgesamt sechs) radial nach innen gerichtete Falten 125.1' bis 125.6' auf. Durch jede Falte 125.1' bis 125.6' ist eine längserstreckte Einstülpung der flexiblen Zylinderwandung 124' in den von ihr berandeten Zylinderinnenraum 120' hinein geformt. Dies ist vor allem anhand der Querschnittsansicht der Figur 9B gut ersichtlich.

Die einzelnen radial nach innen gerichteten (Dehn-)Falten 125.1' bis 125.6' sind hier folglich bezogen auf eine Mittellinie des Zylinders 12' konkav nach innen gewölbt. Die sich hierdurch zwischen einem Paar benachbarter Falten (z. B. 125.6' und 125.1') ergebenden Ausstülpungen, die radial nach außen weisen, sind hier im Querschnitt im Wesentlichen rechteckig. Die flexible Zylinderwandung 124' kann aber alternativ auch derart mit Faltungen versehen sein, dass diese Ausstülpungen andere geometrische Formen aufweisen, z. B. halbkreisförmig oder trapezförmig sind. Über diese Ausstülpungen liegt die Zylinderwandung 124' bevorzugt in dem aus den Figuren 7 bis 9C ersichtlichen (unbelasteten) Zustand des Zylinders 12' an der Innenseite der Zylinderumhüllung 6 (flächig) an.

Wird der Zylinderinnenraum 120' nach bestimmungsgemäßer Positionierung des Zylinders 12' in einem Penis P mit Fluid befüllt, wird die Zylinderwandung 124' auf- bzw. entfaltet, d. h., die einzelnen nach innen gerichteten Faltungen 125.1' bis 125.6' werden radial nach außen in Richtung auf die Zylinderhülle 6 gedrückt. Dies ist in der Figur 9B durch die radial nach außen verlaufenden Pfeile veranschaulicht. Hierdurch dehnt sich folglich der durch die Zylinderwandung 124' definierte Zylinderkern aus, so dass sich dessen Durchmesser und folglich auch das Volumen des Zylinderinnenraums 120' vergrößert.

Durch das nach Außenstülpen der zunächst nach innen gerichteten Faltungen 125.1' bis 125.6' wird dabei insbesondere auch diejenige Faltung 125.6' radial nach außen gedrückt, die die Aufnahme bzw. den Führungskanal 4' für das Führungsmittel 5' definiert. Der Führungskanal 4' ist damit nur temporär in der Zylinderwandung 124' ausgebildet, insbesondere für die Zeit während der bestimmungsgemäßen Positionierung des (unbefüllten) Zylinders 12'.

Im Gebrauch des implantierten Zylinders 12' können sich (die) einzelne(n) Falten 125.1' bis 125.6' zwar wieder einstellen, wenn im Zylinderinnenraum 120' ein geringerer Druck herrscht als bei maximal befülltem Zylinder 12' zur Erzeugung einer Erektion. Die Falten 125.1' bis 125.6' und insbesondere der durch die eine Falte 125.6' gebildete Führungskanal 4' liegen jedoch in jedem Fall nicht starr ausgeformt an in der Zylinderwandung 124' vor, sondern sind in Ihrer Größe abhängig vom Druck in dem Zylinderinnenraum 120' bzw. dem Befüllungszustand des Zylinders 12'

Die eine Falte 125.6', die den Mittelabschnitt 4c' des Führungskanals 4' ausbildet, ist dabei, wie aus der Darstellung der Figur 9A ersichtlich ist, als einzige der Falten 125.1' bis 125.6' bis zu dem Zylinderende 121' hin verlängert, so dass sie in den Endabschnitt 4b' mündet. Die anderen entlang des Umfangs der Zylinderwandung 124' weiterhin angeordneten Falten 125.1' bis 125.5' erstrecken sich nicht durchgängig bis zu dem massiven Zylinderende 121', sondern enden in geringem Abstand hierzu.

Die einzelnen Falten 125.1' bis 125.6' sind weiterhin alle gleichmäßig über den Umfang des Zylinders 12' verteilt, um eine gleichmäßige Dehnung der Zylinderwandung 124' in radialer Richtung zu gewährleisten. Die einzelnen Faltungen bzw. Falten 125.1' bis 125.6' sind folglich entlang einer Umfangslinie quer zur Längserstreckungsrichtung des Zylinders 12' in gleichen Abständen zu den jeweils benachbarten Falten angeordnet.

Wesentliche Besonderheit der Ausführungsvariante der Figuren 7 bis 9C im Unterschied zu dem vorangegangenen Ausführungsbeispiel der Figuren 2 bis 6 ist zusammenfassend das Vorsehen von mindestens einer Faltung in der flexiblen (oder gegebenenfalls sogar elastischen) Zylinderwand 124' eines Zylinderkerns, die einerseits zur Volumenvergrößerung des Zylinderinnenraums 120' bei Befüllung mit einem Fluid, z. B. einer Kochsalzlösung, führt und die andererseits eine temporäre Aufnahme 4' für ein Führungsmittel 5' (oder ein Führungsmittel 5 entsprechend den vorangegangenen Figuren 2 bis 6) bildet. Sind mehrere Faltungen 125.1' bis 125.6' in der Mantelfläche des Zylinderkerns des Zylinders 12' vorgesehen, sind diese dabei vorzugsweise des Weiteren gleichmäßig entlang des Umfangs verteilt, um eine gleichmäßige Dehnung über die gesamte Länge des Zylinderkerns zu erreichen.

Die Figur 9C veranschaulicht ferner, dass in einem zweiten Teilbereich 120b' des Zylinders 12', in dem das Zylinderende 121' angeordnet ist, erneut eine Umlenkung des Führungsmittels 5' bereitgestellt ist. Diese Umlenkung quer zu einer Längserstreckungsrichtung des Zylinders 12 erfolgt hierbei erneut um eine Länge (b/2), die mindestens der Hälfte der Breite bzw. des Durchmessers (b) in diesem Bereich entspricht.

Um das Auffalten der Zylinderwandung 124' in dem von der Zylinderhülle 6 umschlossenen Teilbereich 120a' entsprechend der Figur 9C schon bei geringem Druck zu gewährleisten, ist die Wandstärke der Zylinderwandung 124' entsprechend gering gewählt.

Im Ergebnis kann somit mit dem an den Führungskanal 4, 4' in der Zylinderwandung 124, 124' des Zylinders 12, 12' angepassten Führungsmittel 5, 5' der Zylinder 12, 12' für eine Penisprothese (z. B. eine Penisprothese 1 gemäß der Figur 1) in eine bestimmungsgemäße Position innerhalb des Penis P gedrückt werden. Dabei ist das Führungsmittel 5, 5' lösbar mit dem Zylinder 12, 12' verbunden, so dass das Führungsmittel 5, 5' insbesondere entgegengesetzt zu der Richtung, in die der Zylinder 12, 12' über das Führungsmittel 5, 5' während einer operativen Implantierung gedrückt werden kann, von den Zylinder 12, 12' und damit gegebenenfalls über eine einzige Operationsöffnung im Hautgewebe des Patienten manuell entfernbar.

Sowohl in dem Ausführungsbeispiel der Figuren 2 bis 6 als auch in dem Ausführungsbeispiel der Figuren 7 bis 9C ist das Führungsmittel 5, 5' (mit seinem stabförmigen Mittelabschnitt 5c, 5c') jeweils in der Zylinderwandung 124 bzw. 124' in Längserstreckungsrichtung des Zylinders 12, 12' bis in den Bereich der Zylinderspitze 121.1, 121.1' geführt. Die Zylinderwandung 124, 124' stellt hierbei folglich jeweils eine Aufnahme in Form eines längserstreckten Führungskanals 4, 4' bereit, innerhalb der das Führungsmittel 5, 5' bis in das Zylinderende 121, 121' geführt ist. Ferner ist das Führungsmittel 5, 5' jeweils nach bestimmungsgemäßer Positionierung des Zylinders 12, 12' aus diesem Führungskanal 4, 4' und damit aus dem Zylinder 12, 12' entgegen der Einbringungsrichtung R₁ herausziehbar, wobei das Führungsmittel 5, 5' beim Herausziehen bis zum Austritt aus einer dafür vorgesehen Öffnung 40, 40' des Zylinders 12, 12' innerhalb des Zylinders 12, 12' verläuft. Hierdurch wird nicht nur das operative Einführen des Zylinders 12, 12' in einen Penis P eines Patienten erleichtert, sondern auch ein etwaige Verletzung von Gewebe beim Herausziehen des Führungsmittels 5, 5' nahezu ausgeschlossen.

Die einzelnen Komponenten eines erfindungsgemäßen Zylinders bestehen dabei (wie z.B. auch die übrigen Komponenten einer Penisprothese 1) aus einem biokompatiblen Material oder sind mit einem solchen biokompatiblen Material beschichtet. So kann beispielsweise ein Führungsstab oder Führungsdraht als Führungsmittel 5, 5' aus einem biokompatiblen Material, beispielsweise aus einem mit einer biokompatiblen Beschichtung versehenen Federstahl, hergestellt sein. Es sind jedoch auch andere Materialien bzw. Materialkombinationen denkbar, wobei lediglich entscheidend ist, dass das Führungsmittel 5, 5' eine ausreichende Festigkeit aufweist, so dass ein darauf aufgebrachter Druck sicher in den Zylinder eingeleitet wird bzw. zu dessen Verlagerung führt.

### Bezugszeichenliste

- 1: Penisprothese
- 10: Anschlussbereich
- 12, 12*, 12': Zylinder
- 120, 120': Zylinderinnenraum
- 120.1: Innenraumende
- 120a, 120b: Teilbereich
- 120a', 120b': Teilbereich
- 121, 121', 141: Zylinderende
- 121.1, 121.1': Zylinderspitze
- 122: Kontaktfläche
- 123, 123': Anschlussabschnitt
- 124, 124': Zylinderwandung
- 125.1' - 125.6': (Dehn-)Falte
- 126': Auflagebereich
- 14, 14*: Zylinder
- 16: Pumpeinrichtung
- 18: Reservoir
- 20, 22, 24: Leitung
- 32,34: Anschlussstück
- 320, 340: Anschluss
- 321, 322: Leitungskanale
- 323: Spitze
- 4, 4': Führungskanal (Aufnahme)
- 40, 40': Öffnung
- 4a, 4a': Zuführabschnitt (Kanal)
- 4b, 4b': Endabschnitt (Kanal)
- 4bc: Übergangsabschnitt (Kanal)
- 4c, 4c': Mittelabschnitt (Kanal)
- 5, 5': Führungsmittel
- 50, 50': Griffbereich
- 5a, 5a': Zuführabschnitt (Führungsmittel)
- 5b, 5b': Endabschnitt (Führungsmittel)
- 5bc: Übergangsabschnitt (Führungsmittel)
- 5c, 5c': Mittelabschnitt (Führungsmittel)
- 6: Zylinderhülle
- 63: Anschlussabschnitt
- 61: Hüllenende
- b: Breite des Zylinders
- C: Corpus cavernosum penis (Penisschwellkörper)
- E: Glans penis (Eichel)
- F_{D}: Druckkraft
- F_{R}: Ausziehkraft
- F_{Z}: Zugkraft
- M: Mittellinie
- P: Penis
- R₁: Einbringungsrichtung
- R₂: Ausbringungsrichtung
- S: Skrotum

## Patentansprüche

1. Zylinder für eine Penisprothese, der in den Penis eines Patienten implantierbar und zur Erzeugung einer Erektion des Penis mit Fluid befüllbar ist, wobei der implantierbare Zylinder (12, 12')
- eine äußere Zylinderwandung (124, 124'), die einen mit Fluid befüllbaren Zylinderinnenraum (120, 120') des Zylinders (12, 12') gegenüber einem den Zylinder (12, 12') umgebenden Außenraum begrenzt, aufweist und
- ein Führungsmittel (5, 5') zur bestimmungsgemäßen Positionierung des Zylinders (12, 12') in dem Penis (P) aufweist, mittels dessen der Zylinder (12, 12') in eine bestimmungsgemäße Position innerhalb des Penis (P) gedrückt werden kann und das nach der bestimmungsgemäßen Positionierung von dem Zylinder (12, 12') entfernbar ist,
wobei das Führungsmittel (5, 5') zumindest mit einem Abschnitt (5b, 5bc, 5c; 5b', 5c') in der äußeren Zylinderwandung (124') des Zylinders (12') aufgenommen und mit dem Abschnitt (5b, 5bc, 5c; 5b', 5c') in der äußeren Zylinderwandung (124') entlang einer Längserstreckungsrichtung des Zylinders (12') in einer Aufnahme (4') des Zylinders (12') geführt ist, und
wobei das Führungsmittel (5, 5') lösbar mit dem implantierbaren Zylinder (12') verbunden und zumindest mit einem Abschnitt (5b, 5bc, 5c; 5b', 5c') in der dafür vorgesehenen Aufnahme (4') des Zylinders (12') aufgenommen ist,
**dadurch gekennzeichnet, dass**
die Aufnahme (4') in der äußeren Zylinderwandung (124') dadurch ausgebildet ist, dass die Zylinderwandung (124') eine in Richtung auf den Zylinderinnenraum (120') weisende Faltung (125.6') aufweist und sich ein in der Aufnahme (4') in der Zylinderwandung (124') aufgenommener Abschnitt (5b, 5b') des Führungsmittels (5, 5') im Wesentlichen quer zu einer Längserstreckungsrichtung des Zylinders (12') erstreckt.

2. Zylinder nach Anspruch 1, **dadurch gekennzeichnet, dass** das Führungsmittel (5') entlang einer Ausbringungsrichtung (R₂) von dem Zylinder (12') entfernbar ist, die zu einer Einbringungsrichtung (R₁) unterschiedlich ist, entlang welcher der Zylinder (12') in den Penis (P) des Patienten bestimmungsgemäß einzusetzen ist.

3. Zylinder nach einem der vorhergehenden Ansprüche, **dadurch gekennzeichnet, dass** die Zylinderwandung (124') flexibel ist.

4. Zylinder nach Anspruch 3, **dadurch gekennzeichnet, dass** die flexible Zylinderwandung (124') mindestens eine in Richtung auf den Zylinderinnenraum (120') weisende Faltung (125.1' - 125.6') aufweist, so dass sich das Volumen des Zylinderinnenraums (120') bei einer Befüllung mit Fluid durch Auffalten der Zylinderwandung (124') vergrößern kann.

5. Zylinder nach Anspruch 4, **dadurch gekennzeichnet, dass** der Zylinder (12') eine Zylinderhülle (6) aufweist, die die flexible Zylinderwandung (124') zumindest teilweise umschließt und das Auffalten der flexiblen Zylinderwandung (124') begrenzt.

6. Zylinder nach Anspruch 5, **dadurch gekennzeichnet, dass** die Zylinderhülle (6) die durch eine Faltung (125.6') gebildete Aufnahme (4') für das Führungsmittel (5') zumindest teilweise überdeckt.

7. Zylinder nach Anspruch 6, **dadurch gekennzeichnet, dass** der Zylinder (12') derart ausgebildet ist, dass nach einem Entfernen des Führungsmittels (5, 5') von dem Zylinder (12') und bei Befüllung des Zylinders (12') mit Fluid die einzelnen nach innen gerichteten Faltungen (125.1' - 125.6'), insbesondere diejenige Faltung (125.6'), die die Aufnahme (4') für das Führungsmittel (5, 5') definiert, radial nach außen in Richtung auf die Zylinderhülle (6) gedrückt werden.

8. Zylinder nach einem der vorhergehenden Ansprüche, **dadurch gekennzeichnet, dass** das Führungsmittel (5, 5') zumindest mit einem Abschnitt (5bc, 5b; 5b') in einem in Längserstreckungsrichtung des Zylinder (12') liegenden Endbereich (120b') des Zylinders (12') aufgenommen ist, wobei der implantierte Zylinder (12') bei bestimmungsgemäßer Positionierung innerhalb des Penis (P) über eine Außenseite dieses Endbereichs (120b') am Gewebe des Penis (P) anliegt.

9. Zylinder nach einem der vorhergehenden Ansprüche, **dadurch gekennzeichnet, dass** sich der Abschnitt (5b, 5b') des Führungsmittels (5, 5') mit einer Länge im Wesentlichen quer zur Längserstreckungsrichtung des Zylinders (12') erstreckt, die in derselben Größenordnung liegt wie eine sich im Bereich des Abschnitts (5b, 5b') ergebende Breite (b) des Zylinders (12') senkrecht zur seiner Längserstreckung und/oder die wenigstens halb so groß ist wie diese Breite (b).

10. Zylinder nach einem der vorhergehenden Ansprüche, **dadurch gekennzeichnet, dass** sich das Führungsmittel (5, 5') im Inneren des Zylinders (12') zumindest mit einem Abschnitt (5b, 5b') zwischen einem in Längserstreckungsrichtung des Zylinders (12') liegenden Ende eines mit Fluid befüllbaren Zylinderinnenraums (120') und einem in dieser Längserstreckungsrichtung liegenden Ende (121.1') des Zylinders (12') erstreckt.

11. Zylinder nach einem der vorhergehenden Ansprüche, **dadurch gekennzeichnet, dass** das Führungsmittel (5, 5') einen Griffbereich (50, 50') aufweist, über den das Führungsmittel (5, 5') nach einer bestimmungsgemäßen Positionierung des Zylinders (12') innerhalb des Penis (P) von dem Zylinder (12') entfernbar ist, insbesondere über den das Führungsmittel (5, 5') aus einer dafür vorgesehenen Öffnung (40') an dem Zylinder (12') aus dem Inneren des Zylinders (12') herausziehbar ist.

12. Zylinder nach Anspruch 11, **dadurch gekennzeichnet, dass** der Griffbereich (50) des Führungsmittels (5) in einer Anlieferungsposition des Zylinders (12') an dem Zylinder (12') gehalten und vor der Implantierung des Zylinders (12') in einen Penis (P) von dem Zylinder (12') lösbar ist, so dass über den gelösten Griffbereich (50) das Führungsmittel (5) nach der bestimmungsgemäßen Positionierung des Zylinders (12') in dem Penis (P) vollständig von dem Zylinder (12') entfernbar ist.

13. Penisprothese mit wenigstens einem Zylinder nach einem der vorhergehenden Ansprüche 1 bis 12, der in einen Penis (P) eines Patienten implantierbar und zur Erzeugung einer Erektion des Penis (P) mit Fluid befüllbar ist.

## Claims

1. Cylinder for a penile prosthesis which is implantable in the penis of a patient and is fillable with fluid in order to create an erection of the penis, wherein the implantable cylinder (12, 12')
- has an external cylinder wall (124, 124') which delimits a fluid-fillable cylinder interior (120, 120') of the cylinder (12, 12') with respect to an exterior surrounding the cylinder (12, 12'), and
- has a guide means (5, 5') for properly positioning the cylinder (12, 12') in the penis (P), by means of which the cylinder (12, 12') can be pushed into a proper position within the penis (P) and which is removable from the cylinder (12, 12') after proper positioning,
wherein the guide means (5, 5') is received at least with a portion (5b, 5bc, 5c; 5b', 5c') in the external cylinder wall (124') of the cylinder (12') and is guided with the portion (5b, 5bc, 5c; 5b', 5c') in the external cylinder wall (124') in a longitudinal extension direction of the cylinder (12') in a receptacle (4') of the cylinder (12'), and
wherein the guide means (5, 5') is connected detachably to the implantable cylinder (12') and is received at least with a portion (5b, 5bc, 5c; 5b', 5c') in the receptacle (4'), provided therefor, of the cylinder (12'),
**characterized in that**
the receptacle (4') in the external cylinder wall (124') is formed **in that** the cylinder wall (124') has a fold (125.6') directed towards the cylinder interior (120') and a portion (5b, 5b'), received in the receptacle (4') in the cylinder wall (124'), of the guide means (5, 5') extends substantially transversely to a longitudinal extension direction of the cylinder (12').

2. Cylinder according to Claim 1, **characterized in that** the guide means (5') is removable from the cylinder (12') in an extraction direction (R₂) which is different from an introduction direction (R₁) in which the cylinder (12') is intended to be inserted properly into the penis (P) of the patient.

3. Cylinder according to either of the preceding claims, **characterized in that** the cylinder wall (124') is flexible.

4. Cylinder according to Claim 3, **characterized in that** the flexible cylinder wall (124') has at least one fold (125.1'-125.6') directed towards the cylinder interior (120'), such that the volume of the cylinder interior (120') can increase upon filling with fluid by unfolding of the cylinder wall (124').

5. Cylinder according to Claim 4, **characterized in that** the cylinder (12') has a cylinder sleeve (6) which at least partially surrounds the flexible cylinder wall (124') and limits the unfolding of the flexible cylinder wall (124').

6. Cylinder according to Claim 5, **characterized in that** the cylinder sleeve (6) at least partially covers the receptacle (4'), formed by a fold (125.6'), for the guide means (5').

7. Cylinder according to Claim 6, **characterized in that** the cylinder (12') is formed such that, following removal of the guide means (5, 5') from the cylinder (12') and upon filling of the cylinder (12') with fluid, the individual inwardly directed folds (125.1'-125.6'), in particular that fold (125.6') which defines the receptacle (4') for the guide means (5, 5'), are pushed radially outwards towards the cylinder sleeve (6).

8. Cylinder according to one of the preceding claims, **characterized in that** the guide means (5, 5') is received at least with a portion (5bc, 5b; 5b') in an end region (120b') of the cylinder (12') that lies in the longitudinal extension direction of the cylinder (12'), wherein the implanted cylinder (12'), when positioned properly within the penis (P), rests against the tissue of the penis (P) via an outer side of this end region (120b').

9. Cylinder according to one of the preceding claims, **characterized in that** the portion (5b, 5b') of the guide means (5, 5') extends with a length substantially transversely to the longitudinal extension direction of the cylinder (12'), said length being the same size as a width (b), resulting in the region of the portion (5b, 5b'), of the cylinder (12') perpendicular to the longitudinal extension thereof and/or being at least half this width (b).

10. Cylinder according to one of the preceding claims, **characterized in that** the guide means (5, 5') extends in the interior of the cylinder (12') at least with a portion (5b, 5b') between an end, lying in the longitudinal extension direction of the cylinder (12'), of a fluid-fillable cylinder interior (120') and an end (121.1') of the cylinder (12') that lies in this longitudinal extension direction.

11. Cylinder according to one of the preceding claims, **characterized in that** the guide means (5, 5') has a gripping region (50, 50'), via which the guide means (5, 5') is removable from the cylinder (12') after the cylinder (12') has been positioned properly within the penis (P), in particular via which the guide means (5, 5') is extractable from the interior of the cylinder (12') out of an opening (40'), provided therefor, in the cylinder (12').

12. Cylinder according to Claim 11, **characterized in that** the gripping region (50) of the guide means (5) is held against the cylinder (12') in a delivery position of the cylinder (12') and is detachable from the cylinder (12') before the cylinder (12') is implanted in a penis (P), such that, via the detached gripping region (50), the guide means (5) is removable entirely from the cylinder (12') after the cylinder (12') has been positioned properly in the penis (P).

13. Penile prosthesis having at least one cylinder according to one of the preceding Claims 1 to 12, which is implantable in a penis (P) of a patent and is fillable with fluid in order to create an erection of the penis (P).

## Revendications

1. Cylindre pour une prothèse de pénis, qui peut être implanté dans le pénis d'un patient et qui peut être rempli d'un fluide pour la production d'une érection du pénis, dans lequel le cylindre implantable (12, 12')
- présente une paroi de cylindre extérieure (124, 124'), qui limite une chambre intérieure de cylindre (120, 120') du cylindre (12, 12') qui peut être remplie avec un fluide par rapport à une chambre extérieure entourant le cylindre (12, 12'), et
- présente un moyen de guidage (5, 5') pour le positionnement correct du cylindre (12, 12') dans le pénis (P), au moyen duquel le cylindre (12, 12') peut être poussé dans une position correcte à l'intérieur du pénis (P) et qui peut être retiré du cylindre (12, 12') après le positionnement correct,
dans lequel le moyen de guidage (5, 5') est logé avec au moins une partie (5b, 5bc, 5c; 5b', 5c') dans la paroi de cylindre extérieure (124') du cylindre (12') et est guidé avec la partie (5b, 5bc, 5c; 5b', 5c') dans la paroi de cylindre extérieure (124') le long d'une direction d'extension longitudinale du cylindre (12') dans un logement (4') du cylindre (12'), et
dans lequel le moyen de guidage (5, 5') est relié de façon séparable au cylindre implantable (12') et est logé au moins avec une partie (5b, 5bc, 5c; 5b', 5c') dans le logement (4') du cylindre (12') prévu à cet effet, **caractérisé en ce que** le logement (4') dans la paroi de cylindre extérieure (124') est formé par le fait que la paroi de cylindre (124') présente un pli (125.6') orienté en direction de la chambre intérieure de cylindre (120') et qu'une partie (5b, 5b') du moyen de guidage (5, 5') logée dans le logement (4') dans la paroi de cylindre (124') s'étend essentiellement transversalement à une direction d'extension longitudinale du cylindre (12').

2. Cylindre selon la revendication 1, **caractérisé en ce que** le moyen de guidage (5') peut être retiré du cylindre (12') le long d'une direction de retrait (R₂), qui est différente d'une direction d'introduction (R₁), le long de laquelle le cylindre (12') doit être inséré correctement dans le pénis (P) du patient.

3. Cylindre selon l'une quelconque des revendications précédentes, **caractérisé en ce que** la paroi de cylindre (124') est flexible.

4. Cylindre selon la revendication 3, **caractérisé en ce que** la paroi de cylindre flexible (124') présente au moins un pli (125.1'-125.6') orienté en direction de la chambre intérieure de cylindre (120'), de telle manière que le volume de la chambre intérieure de cylindre (120') puisse augmenter lors d'un remplissage avec un fluide par dépliage de la paroi de cylindre (124').

5. Cylindre selon la revendication 4, **caractérisé en ce que** le cylindre (12') présente une gaine cylindrique (6), qui entoure au moins en partie la paroi de cylindre flexible (124') et qui limite le dépliage de la paroi de cylindre flexible (124').

6. Cylindre selon la revendication 5, **caractérisé en ce que** la gaine cylindrique (6) recouvre au moins en partie le logement (4) formé par un pli (125.6') pour le moyen de guidage (5').

7. Cylindre selon la revendication 6, **caractérisé en ce que** le cylindre (12') est réalisé de telle manière que, après un retrait du moyen de guidage (5, 5') hors du cylindre (12') et lors du remplissage du cylindre (12') avec un fluide, les plis individuels (125.1' - 125.6'), en particulier le pli (125.6') qui définit le logement (4') pour le moyen de guidage (5, 5'), soient poussés radialement vers l'extérieur en direction de la gaine cylindrique (6).

8. Cylindre selon l'une quelconque des revendications précédentes, **caractérisé en ce que** le moyen de guidage (5, 5') est logé au moins avec une partie (5bc, 5b; 5b') dans une région d'extrémité (120b') du cylindre (12') située dans une direction d'extension longitudinale du cylindre (12'), dans lequel le cylindre implanté (12') s'applique, en cas de positionnement correct à l'intérieur du pénis (P), par un côté extérieur de cette région d'extrémité (120b') sur le tissu du pénis (P).

9. Cylindre selon l'une quelconque des revendications précédentes, **caractérisé en ce que** la partie (5b, 5b') du moyen de guidage (5, 5') s'étend essentiellement transversalement à la direction d'extension longitudinale du cylindre (12'), avec une longueur qui est du même ordre de grandeur qu'une largeur (b) du cylindre (12') se produisant dans la région de la partie (5b, 5b') perpendiculairement à son extension longitudinale et/ou qui est au moins à moitié aussi grande que cette largeur (b).

10. Cylindre selon l'une quelconque des revendications précédentes, **caractérisé en ce que** le moyen de guidage (5, 5') s'étend à l'intérieur du cylindre (12') au moins avec une partie (5b, 5b') entre une extrémité, située dans la direction d'extension longitudinale du cylindre (12'), d'une chambre intérieure de cylindre (120') pouvant être remplie par un fluide et une extrémité (121.1') du cylindre (12') située dans cette direction d'extension longitudinale.

11. Cylindre selon l'une quelconque des revendications précédentes, **caractérisé en ce que** le moyen de guidage (5, 5') présente une région de poignée (50, 50'), par laquelle le moyen de guidage (5, 5') peut être retiré hors du cylindre (12') après un positionnement correct du cylindre (12') à l'intérieur du pénis (P), en particulier par laquelle le moyen de guidage (5, 5') peut être retiré hors de l'intérieur du cylindre (12') hors d'une ouverture prévue à cet effet (40') dans le cylindre (12') .

12. Cylindre selon la revendication 11, **caractérisé en ce que** la région de poignée (50) du moyen de guidage (5) est maintenue sur le cylindre (12') dans une position de livraison du cylindre (12') et peut être séparée du cylindre (12') avant l'implantation du cylindre (12') dans un pénis (P), de telle manière que le moyen de guidage (5) puisse être entièrement retiré du cylindre (12'), après le positionnement correct du cylindre (12') dans le pénis (P), au moyen de la région de poignée séparée (50).

13. Prothèse de pénis avec au moins un cylindre selon l'une quelconque des revendications précédentes 1 à 12, qui peut être implantée dans un pénis (P) d'un patient et qui peut être remplie d'un fluide pour la production d'une érection du pénis (P).
